# EUROPÄISCHE PATENTANMELDUNG

(11) **EP 3 585 072 A1**
(43) Veröffentlichungstag der Anmeldung: **25.12.2019**
(21) Anmeldenummer: 19175972.9
(22) Anmeldetag: 22.05.2019
(51) Int. Cl.: H04R 25/00, G08B 21/04

(54) **VERFAHREN ZUM BETRIEB EINES HÖRVORRICHTUNGSSYSTEMS UND HÖRVORRICHTUNGSSYSTEM**

(30) Priorität: 18.06.2018 DE 102018209801
(71) Anmelder: Sivantos Pte. Ltd., Singapore 539775 (SG)
(72) Erfinder: KUKLA, Christoph, 91301 Forchheim (DE); WURZBACHER, Tobias, 90768 Fürth (DE); KECANOVIC, Sandro, 91052 Erlangen (DE)
(74) Vertreter: FDST Patentanwälte

(57) **Zusammenfassung**

Bei einem erfindungsgemäßen Verfahren zum Betrieb eines Hörvorrichtungssystems, das wenigstens eine Hörvorrichtung (1) umfasst, wird verfahrensgemäß mittels eines ersten Sensors (10) der Hörvorrichtung (1) eine erste für eine Ist-Tragesituation charakteristische Messgröße (az,Al) erfasst. Wenn die erste Messgröße (az,Al) und/oder ein daraus abgeleiteter zeitlicher Verlauf wenigstens ein vorgegebenes erstes Kriterium erfüllt, wird darauf geschlossen, dass ein Sturz der Hörvorrichtung (1) vorliegt. Wenn das Vorliegen des Sturzes erkannt wird, wird wenigstens eine weitere Messgröße (T) und/oder ein daraus abgeleiteter zeitlicher Verlauf erfasst, und anhand der wenigstens einen weiteren Messgröße (T) ermittelt, ob die Hörvorrichtung (1) nach dem Sturz am Kopf eines Hörgeräteträgers angeordnet ist.

## Beschreibung

Die Erfindung betrifft ein Verfahren zum Betrieb eines Hörvorrichtungssystems, das wenigstens eine Hörvorrichtung umfasst. Ferner betrifft die Erfindung ein Hörvorrichtungssystem mit wenigstens einer solchen Hörvorrichtung.

Eine Hörvorrichtung dient insbesondere in Form eines Hörhilfegeräts einer Person mit einer Hörminderung dazu, die Hörminderung zumindest teilweise auszugleichen. Dazu umfassen übliche Hörvorrichtungen (insbesondere im Fall von Hörhilfegeräten auch als Hörgeräte bezeichnet) regelmäßig wenigstens ein Mikrofon zur Erfassung von Geräuschen aus der Umgebung sowie einen Signalverarbeitungs-Prozessor, der dazu dient, die erfassten Geräusche zu verarbeiten und insbesondere in Abhängigkeit von der individuellen Hörminderung (insbesondere frequenzspezifisch) zu verstärken und/oder zu dämpfen. Die verarbeiteten Mikrofonsignale werden von dem Signalverarbeitungs-Prozessor an einem Ausgabewandler - meist in Form eines Lautsprechers - zur Ausgabe an das Gehör des jeweiligen Hörgeräteträgers weitergeleitet. Je nach Art der Hörminderung kommen als Ausgabewandler auch sogenannte Knochenleitungshörer oder Cochlea-Implantate zur mechanischen bzw. elektrischen Stimulation des Gehörs zum Einsatz. Unter den Begriff Hörvorrichtung werden aber auch andere Geräte wie beispielsweise Kopfhörer, "hearables", persönliche Geräusch-Verstärkungs-Produkte (auch als "PSAPs" bezeichnet), sogenannte Tinnitus-Masker oder Headsets zusammengefasst.

Im üblichen Gebrauch einer Hörvorrichtung kann diese unter Umständen auch zu Boden fallen (im Folgenden als "stürzen" oder "Sturz" bezeichnet). Da es sich bei einer solchen Hörvorrichtung, insbesondere bei einem Hörhilfegerät meist um ein vergleichsweise kleines Gerät handelt ist eine Sturz-Erkennung zweckmäßig, um beispielsweise den Träger der Hörvorrichtung darauf aufmerksam zu machen, dass die Hörvorrichtung gestürzt ist. Eine andere Situation kann aber auch sein, dass der Träger der Hörvorrichtung ebenfalls und somit mit dieser zusammen stürzt. Da es sich bei Trägern einer Hörvorrichtung, insbesondere einem Hörhilfegerät häufig auch um ältere Menschen handelt, die gegebenenfalls auch hilfsbedürftig sind, ist eine möglichst genaue Kenntnis der tatsächlich vorliegenden Situation wünschenswert. Denn für den Fall, dass der Träger der Hörvorrichtung mit der Hörvorrichtung gestürzt ist und gegebenenfalls Hilfe benötigt, kann schlimmstenfalls selbst nicht für die Hilfe sorgen.

Der Erfindung liegt die Aufgabe zugrunde, einen verbesserten Betrieb eines Hörvorrichtungssystems zu ermöglichen.

Diese Aufgabe wird erfindungsgemäß gelöst durch ein Verfahren zum Betrieb eines Hörvorrichtungssystems mit den Merkmalen des Anspruchs 1. Des Weiteren wird die Aufgabe erfindungsgemäß gelöst durch ein Hörvorrichtungssystem mit den Merkmalen des Anspruchs 20. Vorteilhafte und teils für sich erfinderische Weiterentwicklungen und Ausführungsformen der Erfindung sind in den Unteransprüchen und der nachfolgenden Beschreibung dargelegt.

Das erfindungsgemäße Verfahren dient zum Betrieb eines Hörvorrichtungssystems, das wenigstens eine Hörvorrichtung umfasst. Verfahrensgemäß wird dabei mittels eines ersten Sensors der Hörvorrichtung eine für eine aktuelle Tragesituation (im Folgenden: "Ist-Tragesituation") charakteristische Messgröße erfasst. Wenn die Messgröße und/oder ein daraus abgeleiteter zeitlicher Verlauf wenigstens ein vorgegebenes Kriterium erfüllt, wird darauf geschlossen, dass ein Sturz der Hörvorrichtung vorliegt. Für den Fall, dass das Vorliegen des Sturzes erkannt wird, werden daraufhin wenigstens eine weitere Messgröße und/oder ein aus dieser abgeleiteter zeitlicher Verlauf erfasst. Anhand dieser wenigstens einen weiteren Messgröße wird daraufhin ermittelt, ob die Hörvorrichtung auch nach dem Sturz am Kopf eines Trägers der Hörvorrichtung (im Folgenden kurz als "Hörgeräteträger" bezeichnet) angeordnet ist.

D. h., dass zunächst anhand der ersten Messgröße ermittelt wird, ob die Hörvorrichtung stürzt und anschließend auf die Detektion des Sturzes hin mittels der wenigstens einen weiteren Messgröße geprüft wird, ob der Hörgeräteträger die Hörvorrichtung - beispielsweise vor, während oder nach dem Sturz - verloren hat.

Charakteristisch bedeutet hier und im Folgenden insbesondere, dass die Messgröße eine quantitative Information beinhaltet, die einen eindeutigen Rückschluss auf die Ist-Tragesituation ermöglicht. Die Messgröße kann hierbei einen mit der Ist-Tragesituation verknüpften Wert unmittelbar angeben. Alternativ kann die Messgröße aber auch eine qualitative Information über die Ist-Tragesituation beinhalten.

Durch das vorstehend beschriebene Verfahren wird vorteilhafterweise ermöglicht, die im Zusammenhang mit dem Sturz stehende Tragesituation besser, d. h. insbesondere gegenüber einer "blanken" Detektion des Sturzes der Hörvorrichtung besonders präzise einschätzen zu können. Eine solche präzise Einschätzung kann dabei weiter vorteilhafterweise eine vergleichsweise verlässliche - d. h. mit einem besonders geringen Risiko einer Fehlinterpretation - Entscheidungsgrundlage bezüglich nachfolgend zu treffender oder zu ergreifender Maßnahmen bilden.

In einer besonders bevorzugten Ausführung wird in Abhängigkeit insbesondere von einer Position (Anordnung) der Hörvorrichtung, die die Hörvorrichtung nach dem Sturz einnimmt oder eingenommen hat, eine vorgegebene Maßnahme ergriffen. D. h. es wird zunächst ermittelt, ob die Hörvorrichtung am Kopf des Hörgeräteträgers oder an einer anderen Stelle, beispielsweise am Boden angeordnet ist, und in Abhängigkeit davon gegebenenfalls unterschiedlich reagiert. Mithin ist es möglich, verschiedene Szenarien bei oder nach dem Sturz zu identifizieren, und entsprechend - gegebenenfalls unterschiedlich - darauf zu reagieren. Insbesondere wird also die Position der Hörvorrichtung als ein Merkmal ("feature") herangezogen, um eine entsprechende Maßnahme auszuwählen.

In einer zweckmäßigen Verfahrensvariante wird als erster Sensor ein Beschleunigungssensor herangezogen. Dieser ist vorzugsweise in die Hörvorrichtung integriert. Als Beschleunigungssensor kommt dabei vorzugsweise ein "mehrachsiger", d. h. ein in mehreren Messachsen sensitiver - Beschleunigungssensor zum Einsatz. Vorzugsweise handelt es sich dabei um einen auch als "3D-Beschleunigungssensor" bezeichneten Sensor.

Zweckmäßigerweise wird hierbei als erste Messgröße eine Beschleunigung der Hörvorrichtung erfasst. Als erstes Kriterium für den Sturz wird insbesondere dabei herangezogen, ob ein in der Beschleunigung enthaltener und für die Gravitation indikativer Beschleunigungs-Anteil - insbesondere ein aus dem entsprechenden Messsignal des Beschleunigungssensors ableitbarer Gravitations-Vektor - den Wert null annimmt oder zumindest einen vorgegebenen Schwellwert unterschreitet. Diesem Vorgehen liegt insbesondere die Überlegung zugrunde, dass bei einem freien Fall - unabhängig davon, ob die Hörvorrichtung für sich oder der Hörgeräteträger mit der Hörvorrichtung stürzt - die mittels des Beschleunigungssensors gemessene Gravitation sich im Idealfall vollständig oder zumindest - beispielsweise aufgrund von Messungenauigkeiten - zumindest nahezu aufhebt, da die Hörvorrichtung um den gleichen Wert beschleunigt wird. Mithin kann ein kurzzeitiger "Wegfall" (oder "Abfall") der gemessenen Gravitation ein Indiz für einen Sturz bilden. Für den Schwellwert werden in diesem Fall bspw. Werte nahe null, insbesondere etwa 0,1-0,5 g, vorzugsweise um etwa 0,3 g vorgegeben.

Optional wird in Weiterbildungen der vorstehend beschriebenen Verfahrensvariante außerdem die Dauer des Wegfalls bzw. Abfalls der gemessenen Gravitation berücksichtigt, um dieses Ereignis von beispielsweise einem Sprung oder dergleichen unterscheiden zu können. Beispielsweise wird die übliche Dauer des freien Falls bei einem Sturz anhand der üblichen Fallhöhe - beispielsweise etwa die Körpergröße des Hörgeräteträgers - abgeschätzt. Bei einem Trampolinsprung oder einem Sprung von einem Objekt zum Erdboden dürfte sich die Dauer des jeweiligen freien Falls mit hoher Wahrscheinlichkeit regelmäßig von der üblichen Dauer bei einem Sturz unterscheiden. Als übliche Dauer werden dabei (insbesondere in Abhängigkeit von der Größe des Hörgeräteträgers) Werte von etwa 0,4 bis 0,8 Sekunden, insbesondere von etwa 0,55 bis 0,7 Sekunden angenommen.

In einer weiteren, optional zusätzlichen oder alternativen Verfahrensvariante wird - insbesondere ebenfalls mittels des Beschleunigungssensors - die Beschleunigung der Hörvorrichtung dahingehend (d. h. hinsichtlich des ersten Kriteriums) untersucht, ob ein für einen Aufprall charakteristischer Verlauf der Beschleunigung der Hörvorrichtungen erfasst wird. D. h. ein solcher Aufprall wird als alternatives oder optional weiteres erstes Kriterium herangezogen. Ein solcher Aufprall bei einem Sturz äußert sich in einer erkanntermaßen vergleichsweise abrupten Verzögerung der Hörvorrichtung und somit in einer vergleichsweise starken Beschleunigung mit einer Wirkrichtung entgegen der Richtung der gemessenen Gravitation. Die Verzögerung bei einem bewusst durchgeführten Sprung dürfte im Gegensatz dazu mit hoher Wahrscheinlichkeit weniger hohe Beschleunigungswerte erreichen, da sich der Hörgeräteträger dabei üblicherweise "abfängt", beispielsweise durch Beugen der Gliedmaßen eine sukzessive und möglichst lang dauernde Abfederung der kinetischen Energie vollzieht. Beispielsweise wird zur Detektion eines sturzbedingten Aufpralls ein Schwellwertvergleich oder eine Mustererkennung über einen vergleichsweise längeren Zeitraum - insbesondere über die vorstehend beschriebene übliche Dauer eines Sturzes - durchgeführt.

In einer weiteren zweckmäßigen Verfahrensvariante wird als erster Sensor ein für eine Winkeländerung der Hörvorrichtung sensitiver Sensor (auch als "Lagesensor" bezeichnet) herangezogen. Optional kommt dabei der vorstehend beschriebene Beschleunigungssensor zum Einsatz. Alternativ oder zusätzlich kommt ein als "Gyrosensor" bezeichneter gyroskopischer Sensor zum Einsatz. Weiter alternativ oder zusätzlich kommt ein Magnetfeldsensor zum Einsatz. Eine Kombination von wenigstens zwei der vorstehend beschriebenen Sensoren wird dabei hier und im Folgenden als "inertiale Messeinheit" bezeichnet. In dieser Verfahrensvariante wird für den aus der ersten (oder gegebenenfalls einer weiteren erfassten) Messgröße abgeleiteten Verlauf ein Verlauf einer Winkellage der Hörvorrichtung herangezogen. Diese Winkellage der Hörvorrichtung stellt dabei ein Maß für die Ausrichtung der Hörvorrichtung im Raum dar. Als erstes Kriterium für den Sturz wird in dieser Verfahrensvariante herangezogen, ob ein Muster des Verlaufs der Winkellage der Hörvorrichtung wenigstens einem vorgegebenen Muster hinreichend ähnlich ist. Dieses vorgegebene Muster ist dabei vorzugsweise in einer Speichereinheit der Hörvorrichtung als charakteristisch für den Sturz hinterlegt. Beispielsweise wird als ein vorgegebenes Muster eine zeitlich gesehen langsame Rotation der Hörvorrichtung um eine insbesondere signifikant außerhalb der Hörvorrichtung liegende Achse herangezogen. Eine solche langsame Rotation - d. h. mit einer lediglich geringfügigen Drehbewegung der Hörvorrichtung - lässt dabei einen Schluss auf einen Sturz der Hörvorrichtung gemeinsam mit dem Hörgeräteträger zu. Als weiteres Muster wird optional zusätzlich oder alternativ eine vergleichsweise schnelle Mehrfachdrehung der Hörvorrichtung insbesondere um eine "interne" - d. h. innerhalb der Hörvorrichtung liegende - Achse vorgegeben. Eine Ähnlichkeit mit einem solchen Muster lässt dabei einen Schluss auf einen Sturz der Hörvorrichtung für sich alleine zu, beispielsweise für den Fall, dass diese aus ihrer bestimmungsgemäßen Tageposition am Kopf des Hörgeräteträgers "herausgeschlagen" wird. Im Rahmen dieser Verfahrensvariante wird vorzugsweise ein Mustererkennungsverfahren zum Vergleich des erfassten Verlaufs der Winkellage der Hörvorrichtung und dem oder den gegebenenfalls mehreren Mustern angewendet.

In einer weiteren zweckmäßigen Verfahrensvariante wird als ein insbesondere zusätzliches (zweites) Kriterium für den Sturz herangezogen, ob wenigstens eines der vorstehenden beschriebenen Kriterien innerhalb eines vorgegebenen Zeitfensters, das insbesondere etwa der vorstehend beschriebenen üblichen Dauer eines Sturzes entspricht, beispielsweise eine Dauer von 0,5-1,5 Sekunden aufweist, mit Unterbrechung mehrfach erfüllt wird. Dies lässt insbesondere bei einem "blanken" Sturz der Hörvorrichtung - d. h. der Hörvorrichtung alleine - häufig auftretenden, zumindest teilelastischen Stoß auf eine Art "Springen" der Hörvorrichtung schließen. Beispielsweise kann somit eine zweite Phase eines freien Falls - beispielsweise nach dem vorstehend beschriebenen Aufprall - erfasst werden, die zeitlich deutlich kürzer als die "erste Phase" ist. Entsprechendes gilt auch für die Detektion des vorstehend beschriebenen Aufpralls, der bei einem Springen der Hörvorrichtung auch - zumindest schwächer ausgeprägt - ein zweites oder weiteres Mal auftreten kann. Entsprechendes gilt auch für den vorstehend beschriebenen Verlauf der Winkellage, der nach dem Aufprall der Hörvorrichtung ebenfalls eine zweite Phase einer Rotation der Hörvorrichtung um eine - meist anders ausgerichtete und angeordnete Achse - erfolgt.

In einer weiteren zweckmäßigen Verfahrensvariante wird - alternativ oder optional zusätzlich zu wenigstens einer der vorstehend beschriebenen Verfahrensvarianten - anhand der ersten Messgröße eine aktuelle Ausrichtung (auch: "Ist-Ausrichtung") der Hörvorrichtung ermittelt und diese mit einer Standard-Trage-Ausrichtung verglichen. Optional ist die Standard-Trage-Ausrichtung dabei bei einer Anpassung der Hörvorrichtungen nutzerspezifisch ermittelt und als Referenzwert hinterlegt. Als (optional weiteres) erstes Kriterium für den Sturz wird dabei herangezogen, ob die Ist-Ausrichtung signifikant von der Standard-Tage-Ausrichtung abweicht. Unter signifikanter Abweichung wird dabei hier und im Folgenden insbesondere verstanden, dass die Ist-Ausrichtung sich von einer "leicht" verschobenen Ausrichtung am Kopf des Hörgeräteträgers unterscheiden lässt. Beispielsweise wird als signifikante Abweichung eine Neigung der Hörvorrichtung insbesondere um mehr als zehn Grad um wenigstens eine beliebige Achse herangezogen. In diesem Fall handelt es sich somit vorzugsweise um eine Art Vorher-Nachher-Vergleich der Ist-Ausrichtung, der vorzugsweise fortlaufend oder beispielsweise in vergleichsweise kurzen Zeitintervallen von 0,5-1,5 Sekunden durchgeführt wird. Wird die signifikante Abweichung der Ist-Ausrichtung von der Standard-Tage-Ausrichtung festgestellt, wird auf einen erfolgten Sturz geschlossen. Entspricht die Ist-Ausrichtung der Standard-Trage-Ausrichtung ist mithin die Wahrscheinlichkeit hoch, dass die aktuelle Ist-Trageposition der Hörvorrichtung die bestimmungsgemäße Trageposition (insbesondere der Kopf des Hörgeräteträgers aufrecht gehalten und die Hörvorrichtung in der bestimmungsgemäßen Position getragen wird) darstellt.

In einer weiteren zweckmäßigen Verfahrensvariante wird bei Vorliegen (d. h. Detektion) des Sturzes als weitere Messgröße mittels eines Lautsprechers und eines Mikrofons der Hörvorrichtung eine für eine akustische Rückkopplung charakteristische Rückkopplungs-Größe erfasst und diese, insbesondere ein daraus ableitbares Muster mit einer für einen bestimmungsgemäßen Tragezustand der Hörvorrichtung am Kopf des Hörgeräteträgers hinterlegten Rückkopplungs-Referenzgröße verglichen. Als Rückkopplungs-Größe wird dabei vorzugsweise eine Impulsantwort und/oder eine zugeordnete Frequenzantwort insbesondere des Mikrofons auf ein Testsignal des Lautsprechers herangezogen. Weicht die erfasste Rückkopplungs-Größe um einen vorgegebenes Maß von der Rückkopplungs-Referenzgröße ab, wird entsprechend auf ein Nicht-Vorliegen des bestimmungsgemäßen Tragezustands der Hörvorrichtung am Kopf des Hörgeräteträgers geschlossen.

Anhand dieses Vergleichs und somit auch anhand der erfassten Rückkopplungs-Größe wird dabei in einer vorteilhaften Weiterbildung auf einen Verlust der Hörvorrichtung (vor, bei oder nach dem Sturz) und/oder auf einen Sturz des Hörgeräteträgers gemeinsam mit der Hörvorrichtung geschlossen. Erkanntermaßen lässt sich anhand der Rückkopplungs-Größe, insbesondere anhand der Impulsantwort oder der Frequenzantwort abschätzen, ob sich die Hörvorrichtung bestimmungsgemäß am oder im Ohr des Hörgeräteträgers befindet. Ebenso lässt sich aber anhand der Rückkopplungs-Größe abschätzen, ob sich die Hörvorrichtung zwar bestimmungsgemäß im oder am Ohr des Hörgeräteträgers befindet, dieser aber womöglich - im vorliegenden Fall unter Umständen sturzbedingt - auf diesem Ohr liegt. Letzteres deutet entsprechend auf einen Sturz gemeinsam mit der Hörvorrichtung ohne deren Verlust hin. Optional kann auch ein gemeinsamer Sturz mit einem anschließenden Verlust der Hörvorrichtung erkannt werden, insbesondere indem auf ein Indiz für den Sturz, bspw. bei Detektion des Wegfalls der Gravitation, die Erfassung der Rückkopplungs-Größe vorzugsweise mehrfach nacheinander ausgelöst wird. Dadurch kann anhand einer (ggf. sukzessiven) Veränderung der Rückkopplungs-Größe in Richtung einer für eine vom Ohr separate Anordnung charakteristischen (optional weiteren) Rückkopplungs-Referenzgröße auf den nachträglichen Verlust der Hörvorrichtung geschlossen werden.

In einer bevorzugten Ausführung - insbesondere unabhängig von den vorstehend beschriebenen Varianten der Erfassung der ersten bzw. entsprechenden Messgrößen zur Detektion des Sturzes und/oder der Situation nach dem Sturz - umfasst das Hörvorrichtungssystem zwei Hörvorrichtungen, nämlich vorzugsweise eine rechte und eine linke Hörvorrichtung, die entsprechend zum Tragen am rechten bzw. linken Ohr des Hörgeräteträgers eingerichtet und vorgesehen sind. Vorzugsweise handelt es sich in diesem Fall bei dem Hörvorrichtungssystem um ein binaurales System.

In einer zweckmäßigen Weiterbildung der vorstehend beschriebenen Verfahrensvarianten zur Bestimmung der Rückkopplungs-Größe wird insbesondere für beide Hörvorrichtungen jeweils eine entsprechende Rückkopplungs-Größe erfasst und anhand beider erfassten Rückkopplungs-Größen geprüft, ob der Hörgeräteträger auf einer Seite, insbesondere auf dem entsprechenden Ohr liegt. Dabei werden die beiden (insbesondere rechten bzw. linken) Rückkopplungs-Größen nicht nur mit der (vorzugsweise entsprechend zugeordneten, d. h. insbesondere bei einer Erstanpassung entsprechend hinterlegten) Rückkopplungs-Referenzgröße sondern auch untereinander verglichen. Vorzugsweise wird diese Prüfung - insbesondere wenn bereits festgestellt wurde, dass der Hörgeräteträger auf einer Seite liegt - über einen vorgegebenen Zeitraum von beispielsweise wenigstens 30 Sekunden, optional bis zu 90 oder 120 Sekunden fortgesetzt. Wird über diesen Zeitraum weiterhin festgestellt, dass der Hörgeräteträger auf der Seite liegt, insbesondere liegen bleibt, wird insbesondere darauf geschlossen, dass eine erhöhte Wahrscheinlichkeit dafür vorliegt, dass der Hörgeräteträger sich verletzt hat. Dies wird vorteilhafterweise einer Entscheidung bezüglich der zu ergreifenden Maßnahme zugrunde gelegt.

Zusätzlich oder alternativ zu der vorstehend beschriebenen Prüfung anhand der beiden (d. h. linken und rechten) Rückkopplungs-Größen, ob der Hörgeräteträger auf der Seite liegt, wird eine vergleichbare Prüfung anhand der mittels des Mikrofons der jeweiligen linken und rechten Hörvorrichtung erfassten Umgebungsgeräusche durchgeführt. Insbesondere wird dabei das Frequenzspektrum der jeweiligen erfassten Umgebungsgeräusche abgeleitet und anhand beider Frequenzspektren geprüft, ob für eine der beiden Hörvorrichtungen ein im Vergleich zur anderen Hörvorrichtung gedämpftes Frequenzspektrum der Umgebungsgeräusche erfasst wird. Dies deutet darauf hin, dass das zugeordnete Ohr gegenüber der Umgebung abgedeckt ist und somit beispielsweise nahe dem Boden angeordnet ist oder auf diesem aufliegt. Vorzugsweise wird das mit der jeweiligen Hörvorrichtung erfasste Frequenzspektrum nach dem detektierten Sturz auch mit dem Frequenzspektrum vor dem Sturz verglichen, um beispielsweise auszuschließen dass das Ohr, an dem das vergleichsweise gedämpfte Frequenzspektrum erfasst wird, beispielsweise durch ein Kleidungsstück verdeckt ist. Dadurch kann das Risiko einer Fehlinterpretation verringert werden.

In einer weiteren zweckmäßigen Verfahrensvariante wird zur Ermittlung der weiteren Messgröße ein vorzugsweise in wenigstens einer der gegebenenfalls zwei Hörvorrichtungen eingesetzter Biosensor herangezogen. Die mittels dieses Biosensors erfasste weitere Messgröße oder deren Verlauf wird dabei mit einer für den bestimmungsgemäßen Tragezustand der oder der jeweiligen Hörvorrichtung hinterlegten Referenzgröße bzw. einem entsprechenden Referenzverlauf verglichen. Unter dem Begriff "Biosensor" wird dabei hier und im Folgenden insbesondere ein Sensor verstanden, der als Messgröße eine durch eine Interaktion mit dem Körper des Hörgeräteträgers erfassbare Größe verwendet. Vorzugsweise wird dieser Biosensor dabei dazu genutzt, ein Weiterbestehen der Interaktion des Körpers des Hörgeräteträgers mit der Hörvorrichtung zu detektieren und bei Fehlen einer solchen Interaktion entsprechend ein Indiz für den Verlust der Hörvorrichtung zur Verfügung zu stellen. Insbesondere im Vergleich zu der vorstehend beschriebenen Rückkopplungs-Messung bietet der Biosensor somit eine vergleichsweise einfache und hinsichtlich des erforderlichen Rechenaufwands unaufwändige Möglichkeit, zu bestimmen, ob sich die Hörvorrichtung nach dem Sturz am Kopf des Hörgeräteträgers befindet.

Als Biosensor wird hierbei vorzugsweise ein Temperatursensor, ein Körperschallsensor, ein EKG-Sensor, ein Pulssensor und/oder ein kapazitiver Sensor herangezogen. Bei dem Temperatursensor handelt es sich beispielsweise um ein - auf (Wärme-)Konduktion beruhendes - Widerstandsthermometer. Alternativ handelt es sich bei dem Temperatursensor aber um einen Infrarot-Temperatursensor (kurz als "IR-Temperatursensor" bezeichnet). Dieser weist den Vorteil auf, dass er auf Temperaturänderungen vergleichsweise schnell, d. h. mit einer besonders geringen Zeitverzögerung reagiert. Im Fall des Temperatursensors wird insbesondere als Kriterium für den Verlust der Hörvorrichtung ein Absinken der erfassten Temperatur von der Körpertemperatur herangezogen. Bei den anderen Sensoren wird insbesondere ein fehlendes oder einen nicht sinnhaften Wert anzeigendes Messsignal, beispielsweise ein Fehlen von Körperschall, Fehlen elektrischer Signale bzw. Fehlen eines Pulssignals herangezogen.

Insbesondere, um die Situation des Hörgeräteträgers und/oder der Hörvorrichtung nach dem detektierten Sturz besser analysieren zu können, wird in einer vorteilhaften Verfahrensvariante - nach Detektion des Sturzes - überprüft, ob die aktuelle Ausrichtung - d. h. die Ist-Ausrichtung - der Hörvorrichtung innerhalb eines vorgegebenen Zeitfensters nach dem Sturz verändert wird. Vorzugsweise wird dabei auch überprüft, ob die Ist-Ausrichtung zurück in die Standard-Trage-Ausrichtung verändert wird. Als Zeitfenster wird in diesem Fall beispielsweise wiederum ein Zeitraum von 30, 90 oder 120 Sekunden nach dem Sturz herangezogen. Eine Veränderung der Ist-Ausrichtung der Hörvorrichtung innerhalb dieses Zeitraums wird dabei als Indiz dafür herangezogen, dass - für den Fall eines Verlusts der Hörvorrichtung - der Hörgeräteträger die heruntergefallene Hörvorrichtung wieder aufhebt oder der gemeinsam mit der Hörvorrichtung gestürzte Hörgeräteträger sich mit der Hörvorrichtung am Ohr selbst wieder bewegt. Eine Rücckehr der Hörvorrichtung in ihre Standard-Trage-Ausrichtung wird dabei dahingehend aufgefasst, dass der Hörgeräteträger die Hörvorrichtung wieder angelegt und/oder sich selbst (ggf. mit der nicht-verlorenen Hörvorrichtung) wieder in seine normale Körperhaltung aufgerichtet hat. In beiden Fällen wird vorzugsweise darauf geschlossen, dass der detektierte Sturz unkritisch war. Entsprechend umgekehrt kann darauf geschlossen werden, dass - bei Verlust der Hörvorrichtung vor oder beim Sturz - der Hörgeräteträger die Hörvorrichtung nicht wiedergefunden hat und/oder der zumindest zunächst mit der Hörvorrichtung gestürzte Hörgeräteträger gegebenenfalls bedingt durch den Sturz selbst nicht imstande ist, sich selbst wieder aufzurichten, wenn keine Veränderung der Ist-Ausrichtung detektiert wird.

In einer besonders vorteilhaften Verfahrensvariante wird als vorgegebene Maßnahme ein Notruf abgesetzt, wenn nach dem Sturz die Hörvorrichtung am Kopf des Hörgeräteträgers angeordnet ist und insbesondere festgestellt wird, dass die Hörvorrichtung vorzugsweise innerhalb des vorstehend beschriebenen vorgegebenen Zeitfensters nicht, insbesondere nicht in die Standard-Trage-Ausrichtung zurückbewegt wird. Der Notruf wird dabei vorzugsweise mittels einer Kommunikationsverbindung der Hörvorrichtung übermittelt, optional mittelbar über ein mit der Hörvorrichtung gekoppeltes Smartphone, ein vergleichbares elektronisches Gerät oder "unmittelbar" mittels einer Datenverbindung der Hörvorrichtung zu einem entsprechenden Server oder Serviceprovider. Bspw. wird der Notruf dabei als tatsächlicher Notruf (ggf. in Form einer entsprechenden Textnachricht) an eine Rettungsleitstelle oder dergleichen übermittelt. Zusätzlich oder alternativ wird der Notruf als Nachricht (in Sprach- oder Textform) an eine Kontaktperson des Hörgeräteträgers (bspw. eine verwandte Person, Pflegepersonal, einen betreuenden Arzt oder dergleichen) übermittelt.

Die vorstehend beschriebene Prüfung, ob die Hörvorrichtung bewegt und insbesondere in ihre Standard-Trage-Ausrichtung zurückbewegt wird, wird dabei optional mittels des Beschleunigungssensors und/oder des für die Winkellage sensitiven Sensors (des Lagesensors), beispielsweise der inertialen Messeinheit durchgeführt. Zusätzlich oder alternativ kann auch die vorstehend beschriebene Rücckopplungs-Messung und/oder Analyse des Frequenzspektrums der erfassten Umgebungsgeräusche genutzt werden. Vorzugsweise wird in diesem Fall eine derartige akustische Analyse zur Unterstützung oder Verifizierung der mittels des Beschleunigungssensors und/oder des Lagesensors" erfassten Situation herangezogen.

In einer weiteren Verfahrensvariante wird als vorgegebenen Maßnahme (zusätzlich oder alternativ zu den vorstehend beschriebenen Maßnahmen) insbesondere bei Verlust der Hörvorrichtung eine Position des Sturzes, d. h. insbesondere ein Ort, an dem der Sturz erfolgt ist, erfasst und in von der Hörvorrichtung externen Speichermitteln, beispielsweise einer online-Datenbank oder dem vorstehend beschriebenen Smartphone hinterlegt. Zusätzlich oder alternativ wird vorteilhafterweise auch mittels der verlorenen Hörvorrichtung selbst, der gegebenenfalls zweiten Hörvorrichtung des binauralen Systems und/oder des Smartphones ein Warnsignal ausgegeben, das den Hörgeräteträger auf den Verlust hinweisen soll. Die Hinterlegung der Position des Sturzes in den vorstehend beschriebenen Speichermedien ermöglicht dabei aber auch eine spätere Rückverfolgung und somit gegebenenfalls ein späteres Wiederauffinden der verlorenen Hörvorrichtung.

In einer bevorzugten Verfahrensvariante werden mehrere der vorstehend beschriebenen unterschiedlichen ersten Messgrößen und der entsprechend zugeordneten ersten Kriterien genutzt, um die Detektion des Sturzes zu verbessern, insbesondere eine Fehlinterpretation zu verringern. Optional werden dabei mehrere der ersten Kriterien kumuliert geprüft. Weiter optional können dabei aber auch verschiedene der ersten Messgrößen zur Verifizierung der Detektion des Sturzes mittels einer anderen ersten Messgröße herangezogen werden.

In einer weiteren zweckmäßigen Verfahrensvariante, insbesondere um die Situation nach dem Sturz weiter zu analysieren, werden als vorgegebenen Maßnahme bei Erkennen des Sturzes mittels des Mikrofons der Hörvorrichtung Umgebungsgeräusche aus der Umgebung der Vorrichtung erfasst und auf das Vorliegen von für den Notfall eines Hörgeräteträgers indikativen Lautäußerungen analysiert. Beispielsweise wird dabei mittels einer Sprachanalyseeinheit der Hörvorrichtung analysiert, ob in den erfassten Umgebungsgeräuschen Hilferufe, Husten, schweres Atmen oder dergleichen insbesondere des Hörgeräteträgers selbst enthalten sind. Dies wird vorzugsweise dazu genutzt, um die Entscheidung, einen Notruf abzusetzen, überhaupt zu fällen, oder die vorstehend beschriebene Prüfung, ob die Hörvorrichtung nach dem Sturz bewegt wird, zu unterstützen. Dies kann dahingehend vorteilhaft sein, dass bei einem Verlust der Vorrichtung nach dem Sturz der Hörgeräteträger verletzt in der Nähe der Hörvorrichtung am Boden liegt und gegebenenfalls um Hilfe ruft.

Zusätzlich oder optional alternativ wird in einer weiteren Verfahrensvariante anhand der erfassten Umgebungsgeräusche geprüft, ob bereits auch andere (d. h. dritte) Personen vor Ort sind, ggf. um dem Hörgeräteträger zu helfen. Insbesondere werden die Umgebungsgeräusche dazu auf das Vorhandensein von einer (gegenüber der Stimme des Hörgeräteträgers abweichenden) Stimme einer dritten Person (deren Stimme bei Anwesenheit potentiell in den Umgebungsgeräuschen enthalten ist) analysiert. Falls zumindest eine dritte Person vor Ort ist, wird optional der Notruf (insbesondere der an eine Rettungsleitstelle) unterbunden oder mit der Zusatzinformation versehen, dass bereits eine potentiell helfende Person vor Ort ist.

In einer vorteilhaften Weiterbildung der vorstehend beschriebenen Verfahrensvariante wird eine Spracherkennung auf der erfassten Stimme der dritten Person durchgeführt, um zu erkennen, ob aus deren Sprachäußerungen auf eine erfolgende Hilfeleistung (bspw. in Form von bekannten Anweisungen, die ggf. auch an weitere Personen gerichtet sind) geschlossen werden kann. Ist dies der Fall, wird optional der Notruf unterbunden, da bereits Hilfe erfolgt oder ein Notruf von der helfenden Person bei Bedarf selbst abgesetzt werden kann.

In einer weiteren zweckmäßigen Verfahrensvariante wird als vorgegebene Maßnahme nach Detektion des Sturzes - insbesondere unabhängig davon, ob die Hörvorrichtung alleine oder gemeinsam mit dem Hörgeräteträger gestürzt ist - dieser Sturz in einer Art "Logbuch" registriert. Dieses Logbuch ist dabei optional in der Hörvorrichtung selbst oder in einer vorzugsweise von dem Hersteller der Hörvorrichtung bereitgestellten online-Datenbank implementiert. Bei Überschreiten einer Grenzanzahl für Verlustfälle der Hörvorrichtung werden bspw. enger getaktete Überwachungsmaßnahmen (bspw. die vorstehend beschriebene akustische Rückkopplungs-Prüfung) eingeleitet und/oder der Hörgeräteträger wird angewiesen, den Sitz, insbesondere die Anpassung der Hörvorrichtung überprüfen und ggf. verbessern zu lassen. Zusätzlich oder alternativ wird der Hörgeräteträger bei Überschreiten der Grenzanzahl auch angewiesen, die Hörvorrichtung bspw. von geschultem Personal auf die korrekte Funktion aller Komponenten überprüfen zu lassen, um ggf. aufgetretene Schäden erkennen zu können. Optional weist die Hörvorrichtung selbst auch eine Einrichtung, bspw. einen Controller auf, der eine Systemüberprüfung auf Funktionsfähigkeit aller Komponenten durchführt, wenn die Grenzanzahl überschritten ist - weiter optional auch bei jedem detektierten Verlustfall.

In einer optionalen Verfahrensvariante erfolgt eine Iteration oder nochmalige Überprüfung der zur Detektion des Sturzes herangezogenen Messgrößen bzw. Kriterien, insbesondere wenn sich anhand der zweiten oder weiteren Messgröße ergibt, dass die Hörvorrichtung weiterhin am Ohr des Hörgeräteträgers angeordnet ist. Bspw. wird für den Fall, dass anhand des Abfalls der Beschleunigung in Gravitationsrichtung und/oder anhand der Ist-Ausrichtung der Hörvorrichtung der Sturz der Hörvorrichtung detektiert - oder zumindest die Wahrscheinlichkeit für einen Sturz hochgesetzt wurde - zunächst mittels der Rückkopplungsmessgröße ermittelt, ob die Hörvorrichtung am Ohr des Hörgeräteträgers angeordnet ist. Ergibt die Prüfung der Rückkopplung, dass die Hörvorrichtung am Ohr angeordnet ist, wird in dieser Verfahrensvariante nochmals das Vorliegen des Sturzes nachgeprüft, bspw. anhand des für das Vorliegen des Sturzes ausgegebenen Wahrscheinlichkeitswerts.

Das erfindungsgemäße Hörvorrichtungssystem umfasst wenigstens eine Hörvorrichtung der vorstehend beschriebenen Art. Diese Hörvorrichtung und somit auch das Hörvorrichtungssystem ist dazu eingerichtet, das vorstehend beschriebene Verfahren insbesondere automatisch, d. h. selbsttätig durchzuführen.

Nachfolgend werden Ausführungsbeispiele der Erfindung anhand einer Zeichnung näher dargestellt. Darin zeigen:
- Fig. 1: in einer schematischen Darstellung eine Hörvorrichtung,
- Fig. 2, 3: in einem schematischen Diagramm jeweils eine erste, mittels eines Beschleunigungssenors erfasste Messgröße,
- Fig. 4: in einer schematischen Seitenansicht die Hörvorrichtung in einer Standard-Trage-Ausrichtung am Ohr eines Hörgeräteträgers,
- Fig. 5: die Hörvorrichtung in einer abweichenden Ist-Ausrichtung,
- Fig. 6, 7: jeweils eine Rückkopplungs-Größe für eine außerhalb angeordnete, bestimmungsgemäß oder am Ohr, aber verdeckt getragene Hörvorrichtung,
- Fig. 8: in Ansicht gemäß Fig. 2 einen Verlauf einer Temperatur,
- Fig. 9: in einem schematischen Ablaufdiagramm ein Verfahren zum Betrieb der Hörvorrichtung.

Einander entsprechende Teile und Größen sind in allen Figuren mit gleichen Bezugszeichen versehen

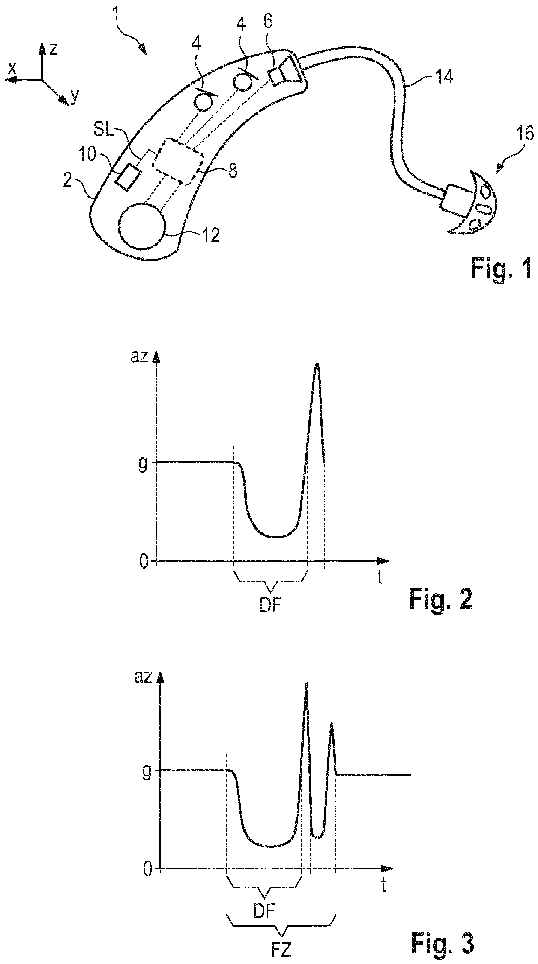

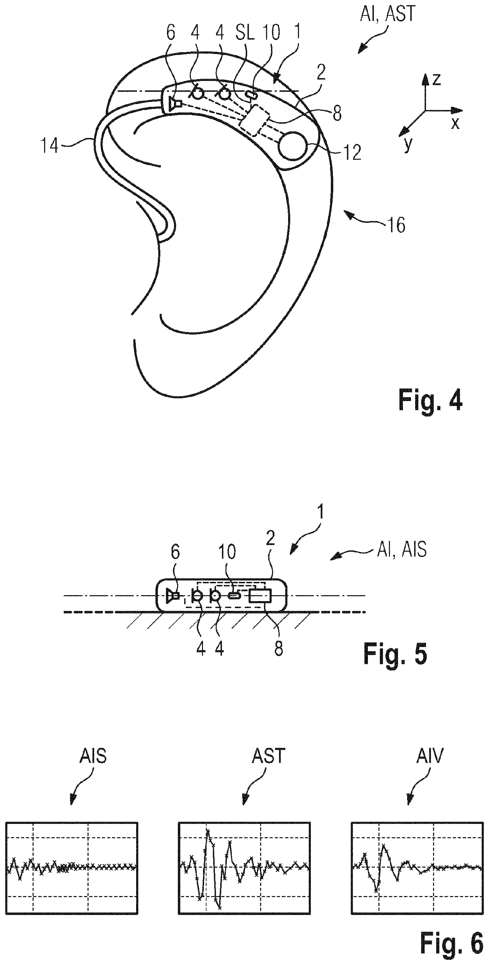

In Figur 1 ist eine Hörvorrichtung, konkret ein Hörhilfegerät (kurz als "Hörgerät 1" bezeichnet) schematisch dargestellt. Das Hörgerät 1 weist ein Gehäuse 2 auf, in dem als elektrische Komponenten des Hörgeräts 1 zwei Mikrofone 4, ein Lautsprecher 6, ein Signalprozessor 8 und ein Lagesensor 10 angeordnet sind. Die Mikrofone 4, der Lautsprecher 6 und der Lagesensor 10 sind signalübertragungstechnisch mit dem Signalprozessor 8 verknüpft. Zur Energieversorgung der elektrischen Komponenten umfasst das Hörgerät 1 außerdem eine Batterie 12, die ebenfalls im Gehäuse 2 angeordnet ist. Zur Schallleitung der von dem Lautsprecher 6 ausgegebenen akustischen Signale umfasst das Hörgerät 1 außerdem einen an dem Gehäuse 2 angekoppelten Schallschlauch 14, der im bestimmungsgemäßen Tragezustand an einem Ohr 16 eines Hörgeräteträgers mit einem Ohrstück 18 in den Gehörgang des Ohrs 16 eingeführt ist (s. Fig. 4).

Der Signalprozessor 8 ist dazu eingerichtet, mittels der Mikrofone 4 empfangene Umgebungsgeräusche trägerspezifisch zu verarbeiten und frequenzspezifisch verstärkt und/oder gedämpft an den Lautsprecher 6 auszugeben. Der Signalprozessor 8 ist außerdem dazu eingerichtet, die aktuelle Tragesituation des Hörgeräts 1 - d. h. ob sich das Hörgerät 1 in seiner in Figur 4 dargestellten Standard-Trage-Ausrichtung AST oder in einer anderen Ausrichtung, konkret überhaupt am Ohr 16 befindet - zu überwachen, konkret einen Sturz des Hörgeräts 1 und/oder des Hörgeräteträgers zu erkennen. Dazu führt der Signalprozessor 8 ein im Folgenden näher beschriebenes Betriebsverfahren durch.

In einem ersten Ausführungsbeispiel ist dabei der Lagesensor 10 als 3D-Beschleunigungssensor- mithin sensitiv in drei unterschiedlichen Raumrichtungen oder Messrichtungen - ausgebildet und bildet dabei einen ersten Sensor des Hörgeräts 1. Aus einem von dem Lagesensor 10 an den Signalprozessor 8 übermittelten Lagesignal SL ermittelt der Signalprozessor 8 eine in vertikaler Richtung gerichtete Beschleunigung az. In einer üblichen Tragesituation (d. h. bei normaler Körperhaltung und in der Standard-Trage-Ausrichtung AST getragenen Hörvorrichtung) entspricht der Wert der Beschleunigung az der Erdbeschleunigung oder Gravitation g. Fällt die gemessene, in vertikaler Richtung gerichtete Beschleunigung az unter einen Schwellwert, der nahe null, konkret bei 0,3 g liegt, ab, folgert der Signalprozessor 8, dass ein freier Fall des Hörgeräts 1 vorliegt und somit das zumindest das Hörgerät 1 fällt oder stürzt. Als (erstes) Kriterium für die Detektion des Sturzes des Hörgeräts 1 zieht der Signalprozessor 8 mithin den Abfall der Beschleunigung az auf null heran. Der zeitliche Verlauf der Beschleunigung az wird mithin vom Signalprozessor 8 als auszuwertendes Merkmal herangezogen.

Zur Reduktion einer Fehlinterpretation von Fluktuationen der Beschleunigung az prüft der Signalprozessor 8, ob sich eine Zeit, für die die Beschleunigung az auf null abfällt, mit einer üblichen Dauer DF eines Sturzes des Hörgeräteträgers oder des Hörgeräts 1 von der Höhe des Ohrs 16 des Hörgeräteträgers deckt (vgl. Fig. 2). Diese Dauer DF wird dabei auf 0,65 Sekunden gesetzt.

In einem alternativen oder optional zusätzlichen Ausführungsbeispiel, das ebenfalls anhand von Figur 2 erläutert wird, wird anhand der Beschleunigung az überprüft, ob der zeitliche Verlauf der Beschleunigung az indikativ für einen Aufprall des Hörgeräts 1 und/oder des Hörgeräteträgers mit dem Hörgerät 1 konkret auf dem Boden ist. Ein solches Indiz liegt vor, wenn die Beschleunigung az Werte aufweist, die für eine abrupte Verzögerung - d. h. entgegen der Gravitation gerichtete Beschleunigung (s. rechter Teil der Kurve, in dem die Kurve über den Wert der Gravitation g überschießt) - aufweist. In Kombination mit dem vorstehend beschriebenen Wegfall der Gravitation g stellt die Überprüfung auf einen solchen, für den Aufprall charakteristischen (oder indikativen) Verlauf der Beschleunigung az ein zusätzliches (erstes) Kriterium für Detektion des Sturzes dar. In Kombination wird hierbei zusätzlich überprüft, ob der für den Aufprall charakteristische Verlauf der Beschleunigung az unmittelbar an den Abfall der Beschleunigung az auf null anschließt.

In einem weiteren, anhand von Figur 3 dargestellten Ausführungsbeispiel überprüft der Signalprozessor 8 als "zweites" Kriterium für den Sturz, ob die Beschleunigung az innerhalb eines vorgegebenen Zeitfenster FZ (beispielsweise 1,2 Sekunden) die beiden vorstehend beschriebenen Kriterien zumindest zweimal nacheinander erfüllt. Ein solches Muster ergibt sich regelmäßig, wenn das Hörgerät 1 für sich alleine zu Boden fällt und dabei vom Boden abspringt. Somit wird bei Erfüllung dieses "zweiten" Kriteriums bereits eine Wahrscheinlichkeit dafür, dass das Hörgerät 1 alleine zu Boden gefallen ist, heraufgesetzt.

In einem weiteren, zusätzlichen oder alternativen Ausführungsbeispiel, dargestellt anhand von Figur 4 und 5, ermittelt der Signalprozessor 8 anhand des Lagesignals SL als Messgröße eine aktuelle Ist-Ausrichtung AI des Hörgeräts 1. Der Lagesensor 10 ist in diesem Ausführungsbeispiel durch eine inertiale Messeinheit gebildet, die den vorstehend beschriebenen 3D-Beschleunigungssensor und einen gyroskopischen Sensor umfasst. Dadurch lässt sich die Ist-Ausrichtung AI des Hörgeräts 1 besonders einfach ermitteln. Der Signalprozessor 8 vergleicht die Ist-Ausrichtung AI mit einer Standard-Trage-Ausrichtung AST (dargestellt in Figur 4). Weicht die Ist-Ausrichtung AI signifikant von der Standard-Trage-Ausrichtung AST ab, schließt der Signalprozessor 8 auf eine hohe Wahrscheinlichkeit, dass das Hörgerät 1 gegebenenfalls zusammen mit dem Hörgeräteträger gestürzt ist. Erkennt der Signalprozessor 8 anhand des Lagesignals SL, dass das Hörgerät 1 - wie in Figur 5 dargestellt - auf einer Seite des Gehäuses 2 liegt (konkret die Ist-Ausrichtung AI um größer als 10 Grad von der Standard-Trage-Ausrichtung AST abweicht), schließt der Signalprozessor 8 darauf, dass das Hörgerät 1 außerhalb des Ohrs 16 angeordnet ist und zumindest nach dem Sturz aus dem Ohr 16 gefallen ist. Diese vom Ohr 16 separate Position oder Ausrichtung des Hörgeräts 1 wird im Folgenden als "separate Ausrichtung AIS" bezeichnet.

Hat der Signalprozessor 8 den Sturz des Hörgeräts 1 gegebenenfalls zusammen mit dem Hörgeräteträger detektiert, ermittelt der Signalprozessor 8 eine weitere Messgröße, um festzustellen, ob das Hörgerät 1 (noch) am Ohr 16 des Hörgerätträgers angeordnet ist. Dazu sendet der Signalprozessor 8 via den Lautsprecher 6 ein Testsignal aus und erfasst dieses mittels der Mikrofone 4 als Rückkopplungssignal. Aus dem erfassten Mikrofonsignal (d. h. dem Rückkopplungssignal) ermittelt der Signalprozessor 8 daraufhin eine Impulsantwort ("impulse response") des Rückkopplungssignals (s. Figur 6). Die Impulsantwort stellt eine Rückkopplungs-Größe dar und wird in diesem Fall als Merkmal herangezogen, um den Tragezustand des Hörgeräts 1 zu ermitteln. Konkret führt der Signalprozessor 8 einen Mustervergleich der erfassten Impulsantwort mit für verschiedene Tragezustände hinterlegten Impulsantworten durch. In Figur 6 ist auf der linken Seite die Impulsantwort für die separate Ausrichtung AIS des Hörgeräts 1 und in der Mitte die Impulsantwort für die Standard-Trage-Ausrichtung AST dargestellt. Auf der rechten Seite der Figur 6 ist die Impulsantwort für ein Szenario dargestellt, in dem das Hörgerät 1 zwar bestimmungsgemäß am Ohr 16 des Hörgeräteträgers angeordnet ist, das Hörgerät 1 aber gegenüber der Umgebung verdeckt ist. Dieser Zustand wird als verdeckte Ausrichtung AIV bezeichnet. Die Impulsantwort für diese verdeckte Ausrichtung AIV ist dabei charakteristisch für die Situation, dass der Hörgeräteträger das Hörgerät 1 beim Sturz nicht verloren hat, aber auf seinem Ohr 16 liegt.

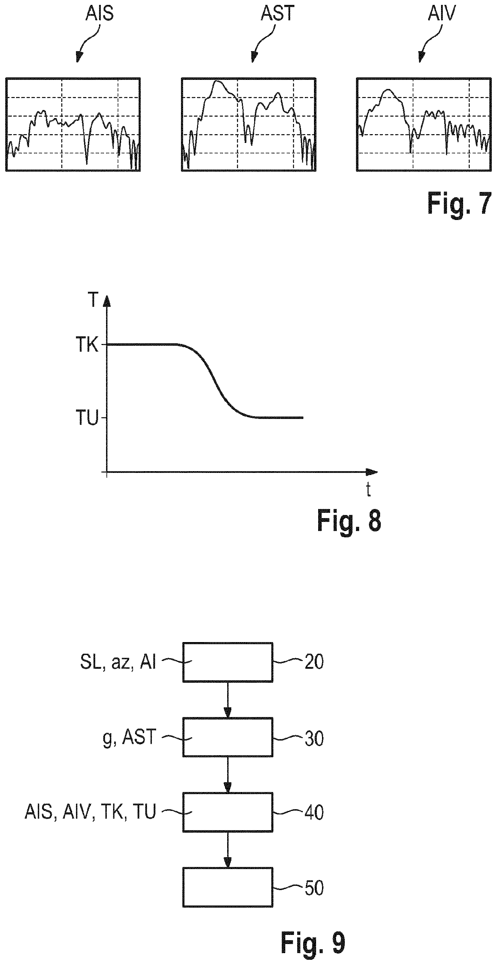

In einem weiteren Ausführungsbeispiel, dargestellt anhand von Figur 7 ermittelt der Signalprozessor 8 anstelle der Impulsantwort eine Frequenzantwort (frequency response) des Rückkopplungssignals. Das Vorgehen gemäß Figur 7 entspricht ansonsten dem gemäß Figur 6.

In einem weiteren, nicht näher dargestellten Ausführungsbeispiel ist das Hörgerät 1 Teil eines binauralen Hörgerätesystems. In diesem Fall werden die Impulsantworten bzw. Frequenzantworten des linken und des rechten Hörgeräts 1 miteinander verglichen. Ist die Impulsantwort bzw. Frequenzantwort des einen Hörgeräts 1 indikativ für die Standard-Trage-Ausrichtung AST, wohingegen die des anderen Hörgeräts 1 für die verdeckte Ausrichtung AIV steht, setzt der Signalprozessor 8 die Wahrscheinlichkeit, dass der Hörgeräteträger ohne Verlust beider Hörgeräte 1 gestürzt ist und auf einem der beiden Ohren 16 liegt, hoch.

Der vorstehend beschriebene Vergleich der Frequenzantworten zwischen beiden Hörgeräten 1 wird in einem weiteren, nicht näher dargestellten Ausführungsbeispiel durch Erfassung der Umgebungsgeräusche mittels beider Hörgerät 1 und Ableitung der jeweiligen Frequenzspektren durchgeführt. Wird dabei für eines der beiden Hörgeräte 1 festgestellt, dass das Frequenzspektrum gegenüber dem anderen Hörgerät 1 gedämpft ist, wird ebenfalls die Wahrscheinlichkeit hoch gesetzt, dass der Hörgeräteträger auf einem der beiden Ohren 16 liegt.

In Figur 8 ist ein weiteres Ausführungsbeispiel dargestellt, wie der Signalprozessor 8 ermittelt, ob das Hörgerät 1 nach dem Sturz separat vom Ohr 16 des Hörgeräteträgers angeordnet ist. Dazu weist das Hörgerät 1 in nicht näher dargestellter Weise einen auch als "Biosensor" bezeichneten Temperatursensor auf. Mittels des Temperatursensors wird die Temperatur T an einer Stelle des Hörgeräts 1 erfasst, an der das Hörgerät 1 in der bestimmungsgemäßen Trageposition mit dem Körper des Hörgeräteträgers in Kontakt steht. Solange das Hörgerät 1 also am Ohr 16 angeordnet ist, entspricht der mittels des Temperatursensors erfasste Temperaturwert somit zumindest etwa einer Körpertemperatur TK. Fällt, während oder nachdem der Sturz festgestellt wird, der erfasste Temperaturwert ab, beispielsweise auf eine Umgebungstemperatur TU, zieht der Signalprozessor 8 dies als Indiz heran, dass das Hörgerät 1 nicht mehr am Körper, konkret am Ohr 16 des Hörgeräteträgers angeordnet ist.

In Figur 9 ist ein schematischer Ablauf des Betriebsverfahrens dargestellt. In einem ersten Verfahrensschritt 20 ermittelt der Signalprozessor 8 anhand des Lagesignals SL die Beschleunigung az und/oder die Ist-Ausrichtung AI. In einem nachfolgenden zweiten Verfahrensschritt 30 ermittelt der Signalprozessor 8 anhand dieser Messgrößen, ob die Beschleunigung az der Gravitation g entspricht oder in vorstehend beschriebener Weise von dieser abweicht, bzw. ob die Ist-Ausrichtung AI von der Standard-Trage-Ausrichtung AST abweicht. Dabei trifft der Signalprozessor 8 die Entscheidung - oder setzt zumindest eine Wahrscheinlichkeit hoch -, ob ein Sturz vorliegt.

In einem Verfahrensschritt 40 ermittelt der Signalprozessor 8 in vorstehend beschriebener Weise, ob das oder das jeweilige Hörgerät 1 noch am Ohr 16 des Hörgeräteträgers angeordnet ist.

In einem weiteren Ausführungsbeispiel ermittelt der Signalprozessor im Verfahrensschritt 40 außerdem, ob sich an der aktuellen Situation des Hörgeräts 1 etwas ändert, indem konkret die Ist-Ausrichtung AI für eine vorgegebene Dauer von einer Minute weiter betrachtet wird. Tritt keine Änderung auf, setzt der Signalprozessor 8 die Wahrscheinlichkeit hoch, dass der Hörgeräteträger das Hörgerät 1 verloren hat und nicht mehr findet, oder dass der Hörgeräteträger aufgrund einer Verletzung nicht in der Lage ist, seine Situation zu ändern. Zur Unterstützung der Entscheidung bezüglich einer Verletzung des Hörgeräteträgers zieht der Signalprozessor 8 die vorstehend beschriebene Analyse der Impuls- bzw. Frequenzantwort bzw. des Frequenzspektrums der Umgebungsgeräusche über einen entsprechend langen, auf den Sturz nachfolgenden Zeitraum heran. Da die Impuls- bzw. Frequenzantwort bzw. das Frequenzspektrum eine Information liefern, ob das Hörgerät 1 noch am Ohr 16 des Hörgeräteträgers angeordnet ist, lässt sich somit vergleichsweise genau abschätzen, ob der Hörgeräteträger mit dem am Ohr 16 angeordneten Hörgerät 1 weiter am Boden liegt, ohne sich wesentlich zu bewegen. Dies würde auf eine vergleichsweise schwere Verletzung des Hörgerätträgers schließen lassen.

In einem weiteren Verfahrensschritt 50 ergreift der Signalprozessor 8 eine für die detektiert die Situation des Hörgeräts 1 bzw. des Hörgeräteträgers vorgegebene Maßnahme. Für den Fall, dass das Hörgerät 1 alleine gestürzt ist, sendet der Signalprozessor 8 mittels einer nicht näher dargestellten Datenschnittstelle eine Information über den Ort des Sturzes an ein in Datenverbindung mit dem Hörgerät 1 stehendes Smartphone des Hörgeräteträgers. Den Ort des Sturzes ermittelt der Signalprozessor 8 optional mittels eines zugeordneten Positionssensors oder alternativ mittels einer von den Smartphone bereitgestellten Ortsinformation.

Für den Fall, dass festgestellt wird, dass der Hörgeräteträger mit dem Hörgerät 1 gestürzt ist und sich auch über einen vergleichsweise langen Zeitraum nicht bewegt, gibt der Signalprozessor 8 mittels des vorstehend beschriebenen Smartphones einen Notruf - beispielsweise an eine Notruf-Leitstelle oder an in dem Smartphone hinterlegte Angehörige des Hörgeräteträgers - aus.

Der Gegenstand der Erfindung ist nicht auf die vorstehend beschriebenen Ausführungsbeispiele beschränkt. Vielmehr können weitere Ausführungsformen der Erfindung von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden.

### Bezugszeichenliste

- 1: Hörgerät
- 2: Gehäuse
- 4: Mikrofon
- 6: Lautsprecher
- 8: Signalprozessor
- 10: Lagesensor
- 12: Batterie
- 14: Schallschlauch
- 16: Ohr
- 18: Ohrstück
- 20: Verfahrensschritt
- 30: Verfahrensschritt
- 40: Verfahrensschritt
- 50: Verfahrensschritt

- AI: Ist-Ausrichtung
- AST: Standard-Trage-Ausrichtung
- AIS: separate Ausrichtung
- AIV: verdeckte Ausrichtung
- az: Beschleunigung
- DF: Dauer
- FZ: Zeitfenster
- g: Gravitation
- SL: Lagesignal
- T: Temperatur
- TK: Körpertemperatur
- TU: Umgebungstemperatur

## Patentansprüche

1. Verfahren zum Betrieb eines Hörvorrichtungssystems, das wenigstens eine Hörvorrichtung (1) umfasst, wobei verfahrensgemäß
- mittels eines ersten Sensors (10) der Hörvorrichtung (1) eine erste für eine Ist-Tragesituation charakteristische Messgröße (az,AI) erfasst wird,
- wenn die erste Messgröße (az,AI) und/oder ein daraus abgeleiteter zeitlicher Verlauf wenigstens ein vorgegebenes erstes Kriterium erfüllt, darauf geschlossen wird, dass ein Sturz der Hörvorrichtung (1) vorliegt,
- wenn das Vorliegen des Sturzes erkannt wird, wenigstens eine weitere Messgröße (T) und/oder ein daraus abgeleiteter zeitlicher Verlauf erfasst wird, und
- anhand der wenigstens einen weiteren Messgröße (T) ermittelt wird, ob die Hörvorrichtung (1) nach dem Sturz am Kopf eines Hörgeräteträgers angeordnet ist.

2. Verfahren nach Anspruch 1,
wobei in Abhängigkeit von einer Position der Hörvorrichtung (1) nach dem Sturz eine vorgegebene Maßnahme ergriffen wird.

3. Verfahren nach Anspruch 1 oder 2,
wobei als erster Sensor ein Beschleunigungssensor (10) herangezogen wird, und wobei als erste Messgröße eine Beschleunigung (az) der Hörvorrichtung (1) erfasst wird, und wobei als erstes Kriterium für den Sturz herangezogen wird, ob ein in der Beschleunigung (az) enthaltener und für die Gravitation (g) indikativer Beschleunigungs-Anteil den Wert null annimmt oder zumindest einen vorgegebenen Schwellwert unterschreitet.

4. Verfahren nach einem der Ansprüche 1 bis 3,
wobei als erster Sensor ein Beschleunigungssensor (10) herangezogen wird, und wobei als Messgröße eine Beschleunigung (az) der Hörvorrichtung (1) erfasst wird, und wobei als erstes Kriterium für den Sturz herangezogen wird, ob ein für einen Aufprall charakteristischer Verlauf der Beschleunigung (az) der Hörvorrichtung (1) erfasst wird.

5. Verfahren nach einem der Ansprüche 1 bis 4,
wobei als erster Sensor ein für eine Winkeländerung der Hörvorrichtung sensitiver Sensor (10) herangezogen wird, und wobei als aus der ersten Messgröße abgeleiteter Verlauf ein Verlauf einer Winkellage der Hörvorrichtung (1) herangezogen wird, und wobei als erstes Kriterium für den Sturz herangezogen wird, ob ein Muster des Verlaufs der Winkellage der Hörvorrichtung (1) wenigstens einem vorgegebenen Muster hinreichend ähnlich ist.

6. Verfahren nach einem der Ansprüche 1 bis 5,
wobei als zweites Kriterium für den Sturz herangezogen wird, ob das erste Kriterium oder wenigstens eines der ersten Kriterien innerhalb eines vorgegebenen Zeitfensters mit Unterbrechung mehrfach erfüllt wird.

7. Verfahren nach einem der Ansprüche 1 bis 6,
wobei anhand der ersten Messgröße eine aktuelle Ausrichtung (AI) der Hörvorrichtung (1) ermittelt und mit einer Standard-Trage-Ausrichtung (AST) verglichen wird, und wobei als Kriterium für den Sturz herangezogen wird, ob die aktuelle Ausrichtung (AI) signifikant von der Standard-Trage-Ausrichtung (AST) abweicht.

8. Verfahren nach einem der Ansprüche 1 bis 7,
wobei bei Vorliegen des Sturzes als weitere Messgröße mittels eines Lautsprechers (6) und eines Mikrophons (4) der Hörvorrichtung (1) eine für eine akustische Rückkopplung charakteristische Rückkopplungs-Größe erfasst wird und die erfasste Rückkopplungs-Größe mit einer für einen bestimmungsgemäßen Tragezustand hinterlegten Rückkopplungs-Größe verglichen wird, und wobei bei einer vorgegebenen Abweichung auf ein Nicht-Vorliegen des bestimmungsgemäßen Tragezustands geschlossen wird.

9. Verfahren nach Anspruch 8,
wobei anhand der erfassten Rückkopplungs-Größe auf einen Verlust der Hörvorrichtung (1) und/oder auf einen Sturz des Hörgeräteträgers gemeinsam mit der Hörvorrichtung (1) geschlossen wird.

10. Verfahren nach Anspruch 8 oder 9,
wobei das Hörvorrichtungssystem eine rechte und eine linke Hörvorrichtung (1) umfasst, und wobei anhand beider für die jeweilige Hörvorrichtung (1) erfassten Rückkopplungs-Größen geprüft wird, ob der Hörgeräteträger auf einer Seite liegt.

11. Verfahren nach einem der Ansprüche 1 bis 10,
wobei das Hörvorrichtungssystem eine rechte und eine linke Hörvorrichtung (1) umfasst, wobei jeweils mittels eines Mikrophons (4) Umgebungsgeräusche erfasst werden, wobei jeweils ein Frequenzspektrum der erfassten Umgebungsgeräusche für beide Hörvorrichtungen (1) ermittelt wird, und wobei anhand der Frequenzspektren geprüft wird, ob der Hörgeräteträger auf einer Seite liegt.

12. Verfahren nach einem der Ansprüche 1 bis 11,
wobei zur Ermittlung der weiteren Messgröße (T) ein Biosensor herangezogen wird, und wobei die weitere Messgröße (T) oder deren Verlauf mit einer für den bestimmungsgemäßen Tragezustand hinterlegten Referenzgröße bzw. einem Referenzverlauf verglichen wird.

13. Verfahren nach Anspruch 12,
wobei als Biosensor ein Temperatursensor, ein Körperschallsensor, ein EKG-Sensor, ein Pulssensor und/oder ein kapazitiver Sensor herangezogen wird.

14. Verfahren nach einem der Ansprüche 7 bis 13,
wobei, wenn das Vorliegen des Sturzes erkannt wird, überprüft wird, ob die aktuelle Ausrichtung (AI) der Hörvorrichtung (1) innerhalb eines vorgegebenen Zeitfensters (FZ) nach dem Sturz verändert wird, insbesondere zurück in die Standard-Trage-Ausrichtung (AST).

15. Verfahren nach einem der Ansprüche 1 bis 14,
wobei als vorgegebene Maßnahme ein Notruf abgesetzt wird, wenn nach dem Sturz die Hörvorrichtung (1) am Kopf des Hörgeräteträgers angeordnet ist und festgestellt wird, dass die Hörvorrichtung (1) nicht bewegt wird.

16. Verfahren nach einem der Ansprüche 1 bis 15,
wobei als vorgegebene Maßnahme bei Verlust der Hörvorrichtung (1) eine Position des Sturzes erfasst und in von der Hörvorrichtung (1) externen Speichermitteln hinterlegt wird und/oder wobei ein Warnsignal ausgegeben wird.

17. Verfahren nach einem der Ansprüche 1 bis 16,
wobei als vorgegebene Maßnahme bei Erkennen des Sturzes mittels des Mikrophons (4) der Hörvorrichtung (1) erfasste Umgebungsgeräusche auf das Vorliegen von für einen Notfall indikativen Lautäußerungen analysiert werden.

18. Verfahren nach einem der Ansprüche 1 bis 17,
wobei als vorgegebene Maßnahme bei Erkennen des Sturzes mittels des Mikrophons (4) der Hörvorrichtung (1) erfasste Umgebungsgeräusche auf das Vorliegen von einer Stimme einer dritten Person analysiert werden.

19. Verfahren nach Anspruch 18,
wobei eine Spracherkennung auf der Stimme der dritten Person durchgeführt wird, um zu erkennen, ob aus deren Sprachäußerungen auf eine erfolgende Hilfeleistung geschlossen werden kann.

20. Hörvorrichtungssystem mit wenigstens einer Hörvorrichtung (1), das dazu eingerichtet ist, das Verfahren nach einem der Ansprüche 1 bis 19 durchzuführen.
